# EUROPEAN PATENT APPLICATION

(11) **EP 2 160 081 A1**
(43) Date of publication of application: **03.03.2010**
(21) Application number: 08015146.7
(22) Date of filing: 27.08.2008
(51) Int. Cl.: H05H 1/24, A61B 18/00

(54) **Non-thermal plasma for wound treatment and associated apparatus and method**

(71) Applicant: Max-Planck-Gesellschaft zur Förderung der Wissenschaften e.V., 80539 München (DE)
(72) Inventor: Morfill, Gregor, 81927 Munich (DE); Steffes, Bernd, 85748 Garching (DE); Shimizu, Tetsuji, 85748 Garching (DE); Pompl, René, 80798 Munich (DE); Nosenko, tetiana, 85748 Garching (DE); Stolz, Wilhelm, 80638 Munich (DE); Isbary, Georg, 80796 Munich (DE); Schmidt, Hans-Ulrich, 82049 Pullach (DE)
(74) Representative: Beier, Ralph

(57) **Abstract**

The invention relates to a non-thermal plasma for treatment of a surface, particularly for the treatment of a wound (1), wherein the plasma comprises a partially ionized carrier gas and at least one additive, which preferably has a sterilizing effect on the treated surface and/or improves the healing of the wound (1). Further, the invention relates to a corresponding apparatus and method.

## Description

### Field of the invention

The invention relates to a non-thermal plasma for treatment of a surface, particularly for the treatment of living tissue and especially for the treatment of wounds.

Further, the invention relates to an apparatus and a corresponding method for providing such a non-thermal plasma, particularly for the treatment of living tissue and especially for the treatment of wounds.

### Background of the invention

The use of non-thermal plasmas for the in-vivo sterilization of wounds is disclosed, for example, in WO 2007/031250 A1 and PCT/EP2008/003568.

However, it is desirable to improve the sterilizing effect of the plasma on wounds thereby improving the wound healing.

### Summary of the invention

Therefore, it is a general object of the invention to improve the sterilizing effect of the plasma and the wound healing in a plasma therapy.

This object is achieved by a novel non-thermal plasma comprising at least one additive, which has a sterilizing effect and/or improves the healing of a wound. Therefore, the plasma according to the invention can be termed as a designer-plasma which is specifically designed for the treatment of biologicial tissue, e.g. wounds, skin, etc., while not harming healthy tissue.

The term sterilization means that the concentration of bacteria is reduced by the plasma, which encompasses a reduction by a factor of 10² (i.e. decontamination), 10⁴ (i.e. disinfection) or 10⁶ (i.e. sterilization).

The additive can be selected from a variety of substances including salts (e.g. sulfates, chlorides), metals, organic substances, inorganic substances and compounds or mixtures of the afore-mentioned substances. Other examples for the additive are biomolecules, proteins and enzymes.

Specifically, the additive can be selected from a group consisting of boron, bromine, thallium, silicon, iron, aluminium, silver, particularly colloidal silver, copper, zinc, manganese, ZnSO₄, K₂, MnO₄, FeSO₄, Ti₂SO₄, iodine, SiO₂, KMnO₄, zinc sulfate, copper-(I)-chloride or copper-(II)-chloride, silver nitrate, silver chloride, manganese-(II)-sulphate, (2-bromine-2-nitrovinyl)-benzole or compounds or mixtures of the afore-mentioned substances.

Other examples of additives are helium, neon, argon, krypton, xenon, nitric oxide, oxygen, hydrogen, sulfur hexafluoride, nitrous oxide, hexafluorethane, methane, carbon fluoride, fluoroform, carbon dioxide, ethanol, air, water or mixtures of these substances.

However, it is essential that the additive has a beneficial effect with regard to the plasma treatment. It is preferred that the additive has a beneficial effect on organic or living tissue. In other words, the additive is preferably health-improving. For example, the additive can be a substance which has a sterilizing effect and/or which improves the wound healing. Therefore, the additive is preferably bactericidal, fungicidal and/or antiviral. However, it is also possible that the additive improves the plasma generation or the plasma application.

It should further be noted that the non-thermal plasma according to the invention can comprise different additives with different properties. For example, a bactericide can be used as first additive and a fungicide can be used as a second additive.

It should further be noted that the additives can be gaseous, solid or liquid.

It should further be noted that the additive in the novel plasma can be a substance which can be activated compared with the starting material. Therefore, the plasma can comprise the additive either in an activated form or in an inactive form. In the further process, the additive can be activated.

It should further be noted that the additive in the plasma can be dissociated or non-dissociated.

Moreover, the additive can be a substance which can be coagulated due to thermal effects or for other reasons. Therefore, the plasma can comprise the substances in a coagulated form or in a non-coagulated form.

Further, the novel plasma can comprise the additive either in an ionized form or in a substantially non-ionized form.

The invention further comprises the novel use of the afore-mentioned non-thermal plasma for the treatment of wounds, living tissue or organic tissue.

Another field of application for the non-thermal plasma according to the invention is the sterilization of a natural or artificial body orifice of a human or animal body and/or for the sterilization of a medical instrument during insertion of the medical instrument through the body orifice into a lumen of the human or animal body, wherein the medical instrument is preferably a catheter. For example, the flow of the plasma can be directed to the body orifice in order to avoid an intrusion of bacteria or other pathogens through the body orifice. Further, the plasma can be directed onto the medical instrument (e.g. a catheter) during the insertion of the instrument into the body so that no pathogens are introduced into the body by the medical instrument.

Further, the plasma according to the invention can be used for the sterilization of transplants, e.g. skin, kidneys, livers, hearts or lungs.

Another field of application for the plasma according to the invention is the treatment of skin diseases or skin disorders.

Further, the plasma according to the invention can be used for the sterilization or treatment of a visceral cavity or lumen of a human or animal body, particularly for the sterilization of an oral cavity or an intestinal cavity.

Finally, the plasma according to the invention can be used for the manufacture of a medicine for the treatment of wounds or biological tissue. In this application, the plasma itself constitutes the medicine which can for example be used for the treatment of skin disorders or skin diseases wherein the wound healing is improved.

Moreover, the invention encompasses an apparatus for providing the afore-mentioned non-thermal plasma, particularly for the treatment of wounds.

The apparatus according to the invention comprises at least one carrier gas source which provides a carrier gas, e.g. argon or ambient air. However, the invention is not restricted to a specific type of carrier gas. Therefore, other types of carrier gases can be used, as well, e.g. helium or nitrogen.

It should further be noted that the novel plasma according to the invention can comprise a mixture of several different carrier gases. Therefore, the apparatus according to the invention can comprise several carrier gas sources providing different carrier gases which are mixed.

Further, the apparatus according to the invention comprises at least one plasma generator for ionizing the carrier gas which is provided by the carrier gas source, so that the plasma generator generates a non-thermal plasma. The plasma generator can be a conventional plasma generator as disclosed, for example, in WO 2007/031250 A1 and PCT/EP2008/003568. However, other types of plasma generators can be used, as well. Further, there can be several plasma generators which can be arranged in series or in parallel.

Moreover, the apparatus of the invention comprises at least one additive source providing the additive. For example, the additive source can be a simple gas cylinder containing the additive in a gaseous form.

Alternatively, the additive source can be a coating of an electrode arrangement in the plasma generator, wherein the coating consists of the additive so that the additive escapes from the coating into the carrier gas. In this embodiment, the plasma generator also forms a mixer which is mixing the additive and the carrier gas.

In another embodiment of the invention, the additive source is a component (e.g. a heated wire or a heatable silver ring), which is coated with the additive so that the additive escapes from the component during operation of the apparatus. For example, the additive can be extracted from the component by heating or sputtering the component. Further, the component may be a massive component consisting of the additive. In this embodiment, the afore-mentioned component forms a mixer mixing the additive and the carrier gas.

It should further be noted that the novel plasma according to the invention can comprise a mixture of several different additives. Therefore, the apparatus according to the invention can comprise several additive sources providing different additives which are mixed.

Finally, the apparatus according to the invention comprises a mixer which is mixing the additive with the non-ionized carrier gas and/or with the ionized plasma. For example, the mixer can be simply a junction of two conduits which are fed by the carrier gas on the one hand and by the additive on the other hand. However, the mixer can also be realized in other ways, which has already been mentioned above.

The mixer generally determines the ratio between the carrier gas and the additive, whereas the plasma generator determines the degree of ionization of the plasma, i.e. the percentage of ionized atoms or molecules. Therefore, the mixer is preferably adjustable in such a way that the ratio between the additive and the carrier gas can be adjusted. Further, the plasma generator is preferably adjustable in such a way that the degree of ionization (i.e. the percentage of ionized atoms or molecules) of the plasma can be adjusted.

In a first embodiment of the invention, the additive is mixed with the non-ionized carrier gas, i.e. before the ionization of the carrier gas. In this embodiment, the mixer is arranged upstream before the plasma generator and mixes the non-ionized carrier gas and the non-ionized additive, so that the plasma generator ionizes a mixture of the carrier gas and the additive.

In a second alternative, the mixer is mixing the additive with the ionized plasma, i.e. after the ionization of the carrier gas. In this alternative, the mixer is arranged downstream after the plasma generator and mixes the ionized carrier gas provided by the plasma generator and the substantially non-ionized additive provided by the additive source.

In a third alternative embodiment, the mixer mixes the ionized carrier gas and the ionized additive. Therefore, the mixer is arranged downstream after the plasma generator(s). For example, there can be a first plasma generator for ionizing the carrier gas and a second additive for ionizing the additive. In such a case, the output of both plasma generators is connected to the mixer so that the mixer is arranged downstream after both plasma generators.

It should further be noted that several different additives can be mixed with the carrier gas and/or with the ionized plasma. Therefore, the apparatus according to the invention can comprise several additive sources for providing the different additives.

Further, several mixers can be provided for mixing the different additives with the non-ionized carrier gas and/or with the ionized plasma.

The plasma generator preferably comprises an electrode arrangement for electrically exciting the carrier gas and, possibly, the additive thereby generating the plasma as disclosed, for example, in WO 2007/031250 A1. Further, the apparatus preferably comprises a high-voltage generator which is connected to the electrode arrangement of the plasma generator.

However, other types of plasma generators are possible, as well. For example, the plasma can be produced by an antenna arrangement or by photo-ionization.

In one embodiment of the invention, the mixer is arranged upstream before the plasma generator so that the plasma generator receives a mixture of the carrier gas and the additive. However, the additive source provides the additive to the mixer discontinuously, so that there are additive-free time intervals during which no additive is provided to the plasma generator, and additive-containing time intervals during which the plasma generator is receiving the additive from the additive source. The discontinuous operation of the additive source can be realized, for example, by providing a controllable valve between the additive source and the plasma generator. In this embodiment, the plasma generator is preferably activated during the additive-free time intervals only, so that the additive is substantially not ionized within the plasma generator although the additive passes through the plasma generator.

In this embodiment, the apparatus preferably comprises a controller, which is controlling both the activation of the plasma generator and the gas flow from the additive source to the plasma generator in such a way that no additive is provided to the plasma generator during activation of the plasma generator.

However, it is alternatively possible that the controller controls the activation of the plasma generator and the gas flow from the additive source to the plasma generator in such a way that the mixing of the additive and the ionization within the plasma generator are temporally overlapping. In this embodiment, the degree of ionization (i.e. the percentage of the ionized atoms or molecules) of the additive is determined by the overlapping time-frame between the time period, in which the plasma generator is activated, on the one hand and the time period during which the additive is provided to the plasma generator, on the other hand. Therefore, it is possible to adjust the degree of ionization (i.e. the percentage of the ionized atoms or molecules) of the additive by adjusting the afore-mentioned overlapping time-frame.

In one embodiment of the invention, the afore-mentioned high-voltage generator of the plasma generator produces a pulse train consisting of pulses which are separated by gaps. This can be achieved by periodically switching the high-voltage generator on and off via a controller. Alternatively, the pulse train can be realized by a switch between the high-voltage generator and the plasma generator, wherein the switch is periodically opened and closed. In this embodiment, the additive can be provided to the plasma generator during the gaps only, so that the additive is substantially not ionized by the plasma generator although the additive passes through the plasma generator.

Further, the apparatus can comprise a UV shield (UV: ultraviolet radiation) which is arranged between the plasma generator and the treated object (e.g. a wound) so that any ultraviolet radiation emitted by the plasma generator is at least partially blocked by the UV shield. Therefore, no UV radiation or only a small fraction of the originally generated UV radiation reaches the treated object.

In this embodiment comprising a UV shield, the mixer can be arranged downstream behind the UV shield so that the additive is added to the plasma downstream behind the UV shield with the result that the additive is not affected by the UV radiation which is generated by the plasma generator.

In another embodiment, a catheter is provided for introducing the plasma through a natural or artificial body orifice into a lumen of a human or animal body. For example, the catheter can be introduced through the mouth into the gullet in order to sterilize the gullet, which might be helpful for the treatment of gullet cancer. In this case, the plasma is designed in such a way that it has a cytotoxic effect in order to inactivate malignent cells.

Finally, the invention also encompasses a method of treating a surface, particularly a wound, which is already apparent from the afore-mentioned description.

It should further be noted that the additive is preferably partially ionized, wherein the degree of ionization (i.e. the percentage of the ionized atoms or molecules) is above 1·10⁻⁹, 2·10⁻⁹, 5·10⁻⁹, 10⁻⁸, 2·10⁻⁸, 5·10⁻⁸, or 10⁻⁷ when measured in the plasma production region. Alternatively, the additive can be substantially not ionized, wherein the degree of ionization (i.e. the percentage of the ionized atoms or molecules) is below 10⁻¹⁵, 10⁻¹⁶, 10⁻¹⁷ or 10⁻¹⁸. It should be noted that the term partially means that there is a fraction of atoms and molecules that is ionized.

It should further be noted that the plasma according to the invention preferably comprises a gas temperature (i.e. the temperature of the atoms or molecules) below +100°C, +75°C, +50°C or +40°C, when measured on the treated surface. Further, the pressure of the plasma preferably equals atmospheric pressure, wherein the pressure is preferably in the range of 800hPa-1.200hPa and more preferably in the range of 900hPa-1.100hPa, when measured on the treated surface. Moreover, the degree of ionization (i.e. the percentage of the ionized atoms or molecules) of the carrier gas is preferably above 1·10⁻⁹, 2·10⁻⁹, 5·10⁻⁹, 10⁻⁸, 2·10⁻⁸, 5·10⁻⁸ or 10⁻⁷ when measured in the plasma production region.

It should further be noted that the plasma can also be applied to the surface (e.g. a wound) in a special low pressure environment below 800hPa.

The invention and its particular features and advantages will become more apparent from the following detailed description considered with reference to the accompanying drawings.

### Brief description of the drawings

- Figure 1: is a schematical representation of an apparatus according to the invention for providing a non-thermal plasma containing an additive which is improving the wound healing,
- Figure 2: is a schematical representation of another embodiment of an apparatus for providing a non-thermal plasma for a wound treatment, wherein both the additive and the carrier gas are ionized and then mixed,
- Figure 3: is a schematical representation of another embodiment of an apparatus for providing a non-thermal plasma for wound treatment, wherein several different additives are mixed with the carrier gas,
- Figure 4: is a schematical representation of an outlet of a plasma source comprising a UV shield blocking ultra-violet radiation,
- Figure 5: is a graphical representation of a modification of the embodiment according to Figure 4, wherein the additive is mixed with the plasma downstream behind the UV shield, and
- Figure 6: is a schematical representation of a catheter for introducing the non-thermal plasma into a lumen of a human body through a body orifice.

### Detailed description of the invention

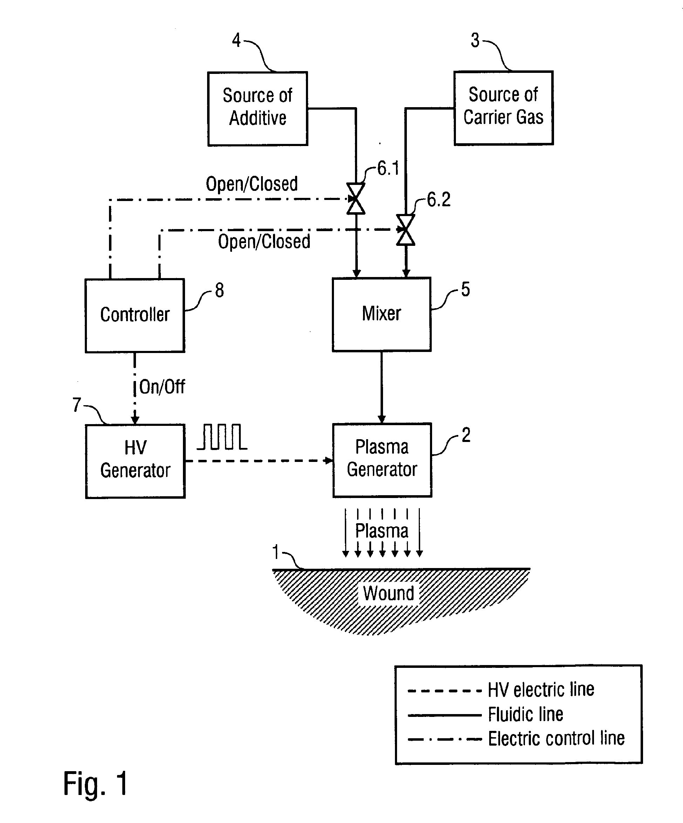

Figure 1 shows a schematical representation of an apparatus for providing an improved non-thermal plasma for the treatment of a wound 1.

The apparatus comprises a plasma generator 2 which can be a conventional plasma generator as disclosed, for example, in WO 2007/031250 A1.

Further, the apparatus comprises a carrier gas source 3 providing a carrier gas, and an additive source 4 providing a gaseous additive which is improving the wound healing.

The additive source 4 is connected to a mixer 5 via a controllable valve 6.1 and the carrier gas source 3 is connected to the mixer 5 via a another controllable valve 6.2.

Therefore, the mixer 5 receives the non-ionized carrier gas (e.g. argon) from the carrier gas source 3 and the non-ionized additive from the additive source 4 and mixes these gases. Then, the mixer 5 provides the mixture of the carrier gas and the additive to the plasma generator 2 which ionizes both the carrier gas and the additive thereby generating the plasma.

The plasma generated by the plasma generator 2 is then applied to the wound 1 wherein the additive has a sterilizing effect on the wound 1 and improves the healing on the wound 1.

It should further be noted that the plasma generator 2 comprises an electrode arrangement for producing the plasma, wherein the electrode arrangement is connected to a high-voltage generator 7 which produces a pulse train consisting of pulses exciting the plasma and gaps between successive pulses. Therefore, the plasma generator 2 ionizes the mixture of the carrier gas and the additive discontinuously in an intermittent on/off-operation with intervals in which the plasma generator 2 is not activated.

Further, the apparatus comprises a controller 8 which controls the pulse train generated by the high-voltage generator 7 and the valves 6.1, 6.2 in such a way that no additive is provided during intervals in which the plasma generator 2 is activated by the high-voltage generator 7. However, the controller 8 opens the valve 6.1 in the intervals during successive pulses of the pulse train generated by the high-voltage generator 7, so that the additive is provided to the plasma generator 2 during the inactive intervals of the plasma generator 2 only. Therefore, the additive is not substantially ionized by the plasma generator 2 although the additive passes trough the plasma generator 2.

Further, the controller 8 controls the ratio between the carrier gas and the additive by controlling the valves 6.1, 6.2 accordingly.

It should further be noted that the high-voltage generator 2 and the valve 6.1 can be controlled in such a way that the active intervals of the plasma generator 2 and the open-intervals of the valve 6.1 are overlapping so that the additive is ionized during the overlapping time interval. Thus, the degree of ionization (i.e. the percentage of ionized atoms or molecules) can be adjusted by adjusting the overlapping time interval.

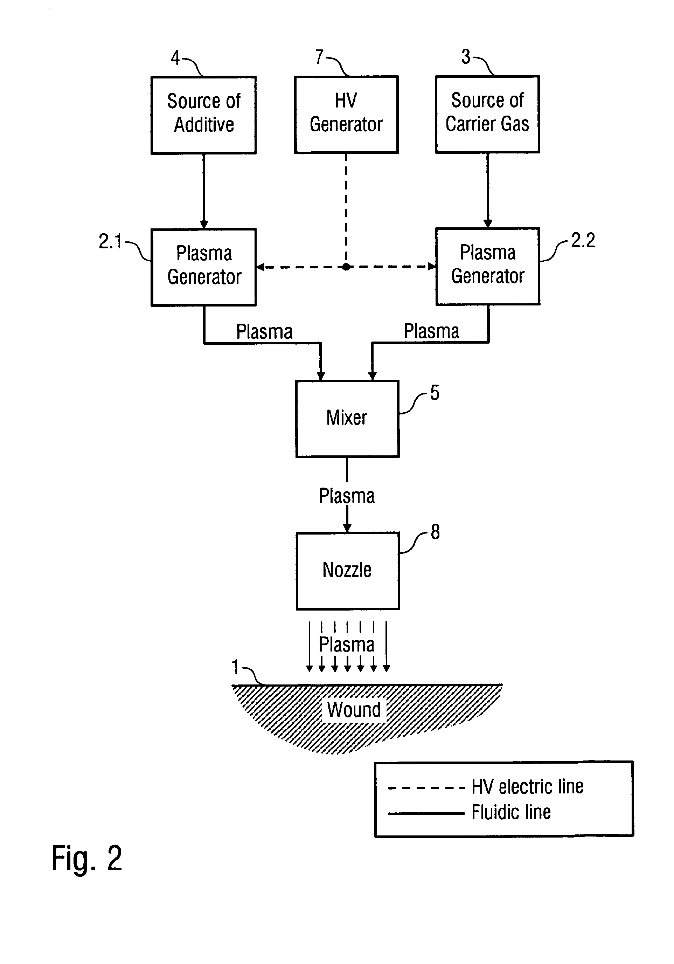

Figure 2 shows a schematical representation of another embodiment of an apparatus for providing an improved non-thermal plasma. The embodiment of Figure 2 is similar to the embodiment of Figure 1 so that reference is made to the above description of Figure 1. Further, the same reference numerals are used for corresponding parts and components.

One characteristic of this embodiment is that both the additive and the carrier gas are ionized separately. Therefore, there are two plasma generators 2.1, 2.2 for ionizing the additive and the carrier gas, respectively.

The plasma generators 2.1, 2.2 are connected to the mixer 5 which is mixing the ionized additive and the ionized carrier gas.

The mixer 5 is in turn connected to a nozzle 8 forming a plasma flow which is directed onto the wound 1 for improving the wound healing.

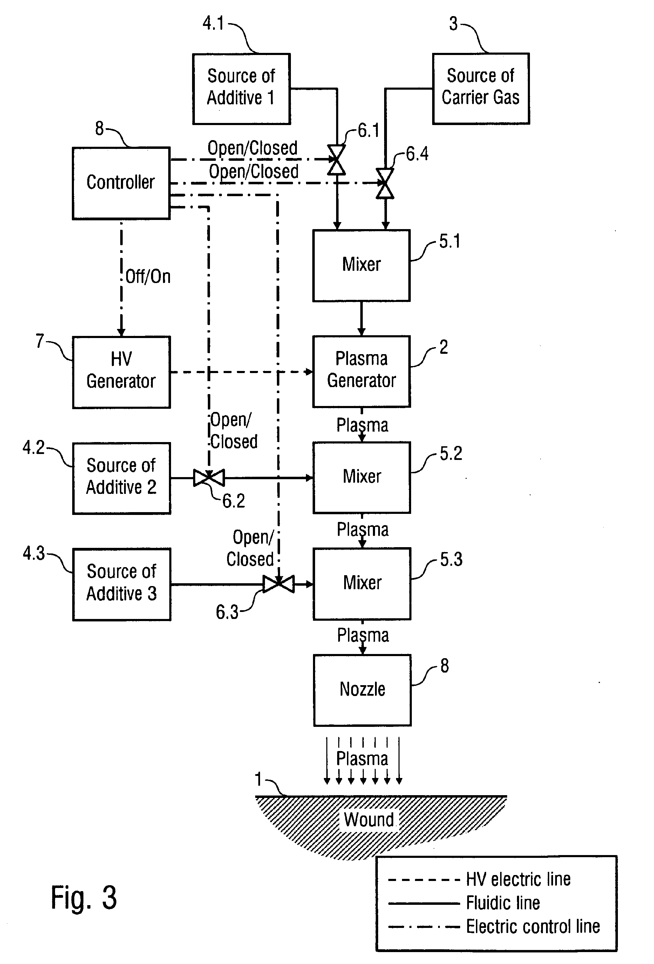

Figure 3 shows a schematic representation of another embodiment of an apparatus for providing a non-thermal plasma wherein this apparatus is similar to the afore-mentioned apparatuses according to Figures 1 and 2 so that reference is made to the above description. Further, the same reference numerals are used for corresponding parts, components and details.

One characteristic of this invention is that the controller 8 actively controls the valves 6.1, 6.2. and 6.3. between the additive sources 4.1, 4.2, and 4.3 and the mixers 5.1, 5.2. and 5.3 and the high-voltage generator 7. The controller 8 also controls the valve 6.4. between the carrier gas source 3 and the mixer 5.1. The controller 8 synchronizes the valves 6.1., 6.4. and the high-voltage generator 7 in such a way that the time period during which the additive from the source 4.1. passes through the plasma generator 2 and the time period during which the carrier gas passes through the plasma generator 2 have a different temporal overlap with the time period during which the plasma generator 2 is activated. Therefore, the degrees of ionization (i.e. the percentage of the ionized atoms or molecules) of the additive from the source 4.1. and of the carrier gas are different and can be tuned independently of each other.

In this embodiment, two further additive sources 4.2, 4.3 are provided which are delivering different additives to mixers 5.2, 5.3 via valves 6.2, 6.3, wherein the mixers 5.2, 5.3 are arranged downstream behind the plasma generator 2 so that the plasma generator 2 does not ionize the additives provided by the additive sources 4.2, 4.3.

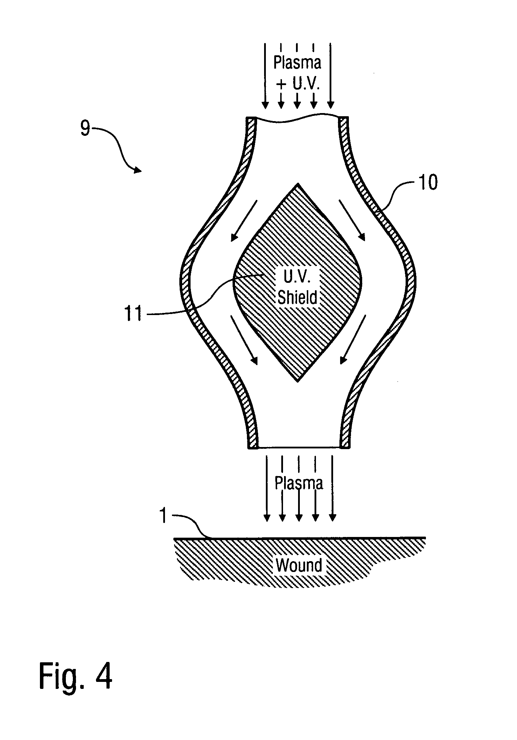

Figure 4 shows a schematical representation of an outlet 9 of the apparatus according to the invention, wherein the outlet 9 applies the plasma to the wound 1.

The outlet 9 essentially consists of an outlet tube 10 guiding the plasma wherein ultraviolet radiation coming from the plasma generator enters the outlet tube 10, as well.

Therefore, the outlet 9 comprises a UV shield 11 which is arranged in the middle of the outlet tube 10 in a bulge of the outlet tube 10 so that the plasma flows around the UV shield 11. The UV shield 11 consists of a UV blocking material (e.g regular window glass) and therefore blocks the ultraviolet radiation entering the outlet tube 10. Therefore, substantially no ultraviolet radiation leaves the outlet 9 so that the wound 1 is not affected by any ultraviolet radiation or only by a small fraction.

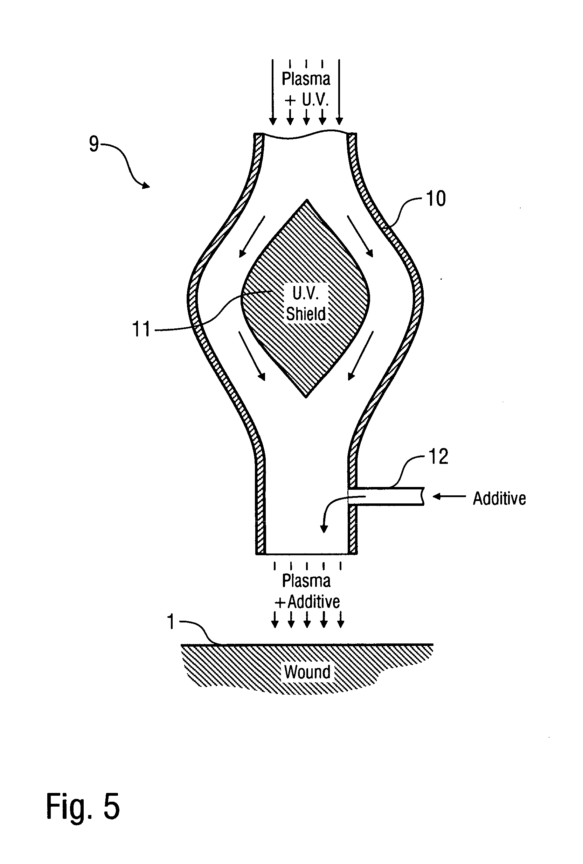

The embodiment of Figure 5 is very similar to the embodiment of Figure 4 so that reference is made to the above description with regard to Figure 4. Further, the same reference numerals are used for corresponding parts, components and details.

One characteristic of this embodiment is that a conduit 12 discharges into the outlet tube 10 downstream behind the UV shield 11 wherein the conduit 12 delivers an additive to the plasma flow within the outlet tube 10. Therefore, the UV shield 11 prevents the additive from being affected by the ultraviolet radiation entering the outlet 9.

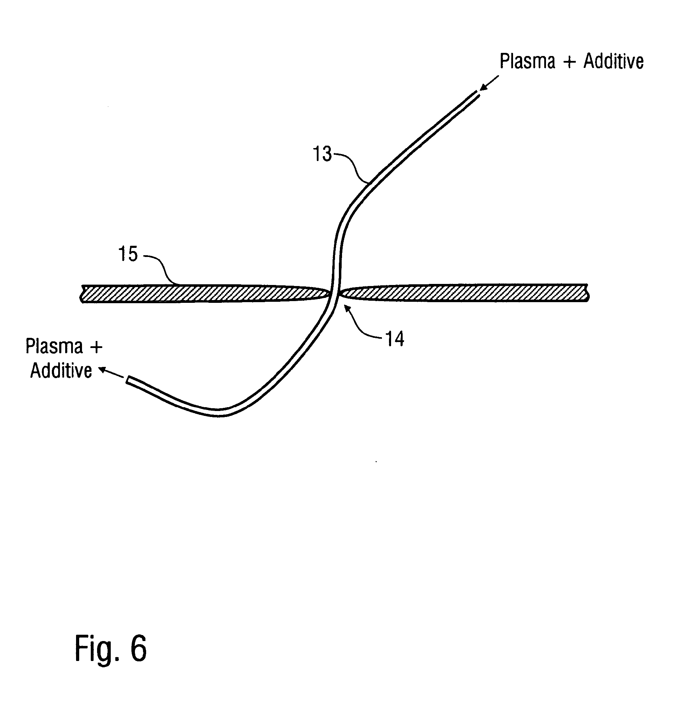

Finally, Figure 6 shows a schematical representation of a hollow catheter 13 which can be inserted into a lumen of a human body through an artificial or natural body orifice 14 in body surface 15. For example, the body orifice 14 can be the mouth of a human being so that the catheter 13 is introduced into the gullet where the catheter 13 can apply the non-thermal plasma comprising the sterilizing additive to the gullet.

Although the invention has been described with reference to the particular arrangement of parts, features and the like, these are not intended to exhaust all possible arrangement of features, and indeed many other modifications and variations will be ascertainable to those of skill in the art.

### List of reference numerals:

- 1: Wound
- 2: Plasma generator
- 3: Carrier gas source
- 4: Additive source
- 4.1: Additive source
- 4.2: Additive source
- 4.3: Additive source
- 5: Mixer
- 5.1: Mixer
- 5.2: Mixer
- 5.3: Mixer
- 6.1: Valve
- 6.2: Valve
- 6.3: Valve
- 6.4: Valve
- 7: High-voltage generator
- 8: Nozzle
- 9: Outlet
- 10: Outlet tube
- 11: U.V. shield
- 12: Conduit
- 13: Catheter
- 14: Body orifice
- 15: Body surface

## Claims

1. Non-thermal plasma for treatment of a surface, particularly for the treatment of biological tissue, especially for the treatment of a wound (1), wherein the plasma comprises a partially ionized carrier gas, **characterized by** at least one additive, which preferably has a sterilizing effect on the treated surface and/or a beneficial effect on the biological tissue and/or which improves the healing of the wound (1).

2. Non-thermal plasma according to claim 1, wherein the additive is selected from a group consisting of:
a) Sulfates
b) Chlorides
c) Salts
d) Metals
e) Organic substances
f) Inorganic substances
h) Biomolecules
i) Proteins
j) Enzymes
k) Compounds or mixtures of the afore-mentioned substances.

3. Non-thermal plasma according to one of the preceding claims, wherein the additive is selected from a group consisting of:
a) Boron
b) Bromine
c) Thallium
d) Silicon
e) Iron
f) Aluminium
g) Silver, particularly colloidal silver
h) Copper
i) Zinc
j) Manganese
k) ZnSO₄
l) K₂MnO₄
m) FeSO₄
n) Ti₂SO₄
o) Iodine
p) SiO₂
q) KMnO₄
r) Zinc Sulfate
s) Copper(I) chloride or Copper(II) chloride
t) Silver nitrate
u) Silver chloride
v) Manganese(II) sulfate
w) (2-bromin-2-nitrovinyl)benzole
y) Helium
z) Neon
aa) Krypton
ab) Xenon
ac) Nitric oxide
ad) Oxygen,
ae) Hydrogen
af) Sulfur hexafluoride
ag) Nitrous oxide
ah) Hexafluorethane
ai) Methane
aj) Carbon fluoride
ak) Fluoroform
al) Carbon dioxide
am) Ethanol
an) Air
ao) Water
ap) Argon
aq) Compounds or mixtures of the afore-mentioned substances.

4. Non-thermal plasma according to one of the preceding claims, wherein
a) the additive is a substance which can be either inactive or activated, wherein the plasma comprises the additive substantially in an inactive form, or
b) the additive is a substance which can be either inactive or activated, wherein the plasma comprises the additive substantially in an activated form, and/or
c) the additive is a substance which can be either dissociated or non-dissociated, wherein the plasma comprises the additive substantially in a dissociated form, or
d) the additive is a substance which can be either dissociated or non-dissociated, wherein the plasma comprises the additive substantially in an non-dissociated form, and/or
e) the additive is a substance which can be either coagulated or non-coagulated, wherein the plasma comprises the additive substantially in a coagulated form, or
f) the additive is a substance which can be either coagulated or non-coagulated, wherein the plasma comprises the additive substantially in a non-coagulated form, and/or
g) the additive is a substance which can be either ionized or non-ionized, wherein the plasma comprises the additive substantially in an ionized form, or
h) the additive is a substance which can be either ionized or non-ionized, wherein the plasma comprises the additive substantially in a non-ionized form.

5. Use of the non-thermal plasma according to any of the preceding claims for
a) the treatment of wounds (1), or
b) the treatment of living tissue, or
c) the treatment of organic tissue, or
d) the sterilization of a natural or artificial body orifice (14) of a human or animal body and/or for the sterilization of a medical instrument during insertion of the medical instrument through the body orifice (14) into a lumen of the human or animal body, wherein the medical instrument is preferably a catheter (13), or
e) sterilization of transplants, or
f) the treatment of skin diseases or skin disorders, or
g) any medical treatment, or
h) the sterilization or treatment of a visceral cavity or lumen of a human or animal body, particularly for the sterilization of an oral cavity or an intestinal cavity, or
i) the manufacture of a medicine for the treatment of wounds (1) or biological tissue.

6. Apparatus for providing a non-thermal plasma, particularly for the treatment of a wound (1), comprising:
a) A carrier gas source (3) providing a carrier gas,
b) a plasma generator (2, 2.1, 2.2) for ionizing the carrier gas provided by the carrier gas source (3) thereby generating the plasma,
**characterized by**
c) an additive source (4, 4.1, 4.2, 4.3) providing an additive and
d) a mixer ( 5, 5.1, 5.2, 5.3) which is mixing the additive with the non-ionized carrier gas and/or with the ionized plasma.

7. Apparatus according to claim 6, wherein
a) the mixer is arranged upstream before the plasma generator and mixes the non-ionized carrier gas and the non-ionized additive, so that the plasma generator ionizes a mixture of the carrier gas and the additive, or
b) the mixer (5, 5.2, 5.3) is arranged downstream behind the plasma generator (2, 2.1, 2.2) and mixes the ionized carrier gas provided by the plasma generator (2, 2.1, 2.2) and the non-ionized additive provided by the additive source (4, 4.2, 4.3).

8. Apparatus according to any of claims 6 to 7, further comprising:
a) several additive sources (4.1, 4.2, 4.3) each providing a different additive, and/or
b) several mixers (5.1, 5.2, 5.3) for mixing the different additives with the non-ionized carrier gas and/or with the ionized plasma, and/or
c) several plasma generators (2.1, 2.2).

9. Apparatus according to any of claims 6 to 8, wherein
a) the plasma generator (2, 2.1, 2.2) comprises an electrode arrangement or an antenna arrangement for electrically exciting the carrier gas thereby generating the plasma, and
b) a high-voltage generator (7) is connected to the electrode arrangement or the antenna arrangement.

10. Apparatus according to claim 9, wherein
a) the mixer (5, 5.1) is arranged upstream before the plasma generator (2) so that the plasma generator (2) receives a mixture of the carrier gas and the additive,
b) the additive source (4, 4.1) provides the additive to the mixer (5, 5.1) discontinously, so that there are additive-free intervals during which no additive is provided to the plasma generator (2),
c) the plasma generator (2) is activated during the additive-free intervals only, or
d) the ionization and the mixing of the additive temporally overlap so that the additive is partially ionized during the overlapping time period.

11. Apparatus according to claim 10, further comprising a controller (8) which is controlling the activation of the plasma generator (2) and the gas flow from the additive source (4, 4.1) to the plasma generator (2) in such a way that
a) no additive is provided to the plasma generator (2) during activation of the plasma generator (2), or
b) the ionization and the mixing of the additive temporally overlap so that the additive is partially ionized during the overlapping time period.

12. Apparatus according to any of claims 9 to 11, wherein
a) the high-voltage generator (7) produces pulses which are separated by gaps,
b) the additive is provided to the plasma generator (7) during the gaps only so that the additive is substantially not ionized.

13. Apparatus according to any of claims 9 to 12, wherein
a) the electrode arrangement of the plasma generator (2, 2.1, 2.2) is at least partially covered with a coating or consisting of the additive so that the additive escapes from the coating into the carrier gas, or
b) the additive source comprises a component consisting of the additive or being covered with the additive so that the additive escapes from the component, wherein the component is preferably heatable to extract the additive from the component.

14. Apparatus according to any of claims 6 to 13, wherein
a) a UV shield (11) is arranged between the plasma generator (2, 2.1, 2.2) and the treated object (1) so that any UV radiation emitted by the plasma generator (2, 2.1, 2.2) is at least partially blocked by the UV shield (11) and does not reach the object (1) or only a small fraction of it, and/or
b) the mixer (10, 12) is arranged downstream behind the UV shield (11) so that the additive is added to the plasma downstream behind the UV shield (11) and the additive is not affected by the UV radiation which is generated by the plasma generator (2, 2.1, 2.2), and/or
c) a catheter (13) is provided for introducing the plasma through a natural or artificial body orifice (14) into a lumen of a human or animal body.

15. Method of treating an object (1), particularly organic tissue and/or living tissue, especially a wound (1), comprising the following steps:
a) Providing a carrier gas,
b) Ionizing the carrier gas thereby generating a plasma,
c) Applying the plasma to the object (1),
**characterized by** the following step:
d) Mixing the carrier gas and/or the plasma with an additive before applying the plasma to the object (1).

16. Method according to claim 15, wherein
a) the additive is mixed with the carrier gas upstream before the ionization, or
b) the additive is mixed with the ionized carrier gas downstream behind the ionization so that the additive is not ionized.

17. Method according to any of claims 15 to 16, wherein
a) the additive is mixed with the carrier gas upstream before the ionization of the carrier gas, and/or
b) the mixing of the additive and the ionization do not overlap temporally so that the additive is substantially not ionized, or
c) the ionization and the mixing of the additive temporally overlap so that the additive is partially ionized during the overlapping time period.

18. Method according to any of claims 15 to 17, further comprising the following step:
a) Mixing several different additives with the carrier gas and/or with the plasma, and/or
b) Introducing the plasma through a natural or artificial body orifice (14) into a lumen of a human or animal body for treatment of the lumen.
